# EUROPÄISCHE PATENTSCHRIFT

(11) **EP 2 392 296 B1**
(45) Veröffentlichungstag und Bekanntmachung des Hinweises auf die Patenterteilung: **05.09.2012**
(21) Anmeldenummer: 10005715.7
(22) Anmeldetag: 02.06.2010
(51) Int. Cl.: A61F 5/00, A61B 17/00

(54) **Implantierbare magenvolumenreduzierende Einrichtung**
Implantable device for reducing stomach volume
Dispositif implantable réduisant le volume gastrique

(43) Veröffentlichungstag der Anmeldung: 07.12.2011
(73) Patentinhaber: AMI Agency for Medical Innovations GmbH, 6800 Feldkirch (AT)
(72) Erfinder: Egle, Walter, 6842 Koblach (AT)
(74) Vertreter: Hofmann, Ralf U.

(56) Entgegenhaltungen:
- WO-A2-2007/017880
- US-A1- 2003 158 569
- US-A1- 2004 059 289
- US-A1- 2006 271 088

## Beschreibung

Fettleibigkeit stellt ein Hauptproblem zur Erhaltung der Gesundheit der Menschheit dar und hat in vielen Ländern der Welt bereits epidemische Ausmaße angenommen. So sind zum Beispiel in den USA 30% der Bevölkerung bereits als "fettleibig" zu bezeichnen und weitere 35% leiden an Übergewicht. Die entsprechenden Vergleichszahlen im Rest der Welt sind den USA-Zahlen sehr nahe, wie z.B. in Europa, Latein Amerika und selbst in Ländern der sogenannten Dritten Welt stark zunehmend. Zur Behandlung der krankhaften Fettleibigkeit haben sich weltweit verschiedene, teilweise sehr invasive chirurgische Verfahren, wie z.B. die Magenbypassoperation etabliert. Bei diesen chirurgischen Verfahren wird durch Magenverkleinerung einerseits versucht das Volumen der zugeführten Nahrungsaufnahme pro Essen zu reduzieren und gleichzeitig durch das Verkürzen des Dünndarmes die Transitzeit der Nahrung durch den Körper verkürzt. Um zu verhindern dass Patienten vom Zustand der Fettleibigkeit in den Zustand der krankhaften Fettleibigkeit gleiten, wird seit Jahrzehnten mit sogenannten Magenballons versucht das Aufnahmevolumen des Magens und damit das Volumen der möglichen Nahrungsaufnahme pro Mahlzeit zu reduzieren.

US 4,694,827 zeigt die Ausführung eines segmentierten Mehrkammerbalions, welcher über die Speiseröhre in den Magen eingebracht wird, wobei die segmentierte Form der Ausführung die Kontaktfläche der Magenschleimhaut, der Mageninnenwand mit dem Ballon reduzieren soll. Die Ausführung kann in einer der Magenanatomie ähnlichen, länglichen Form erfolgen, wobei der Ballon aus flexiblem, dauerhaftem, Pepsin und Magensäure widerstandsfähigem, elastischem Material gefertigt ist, welches gasundurchlässig ist, damit die sich im gefüllten Ballon befindliche und unter Druck stehende Luftblase nicht in den Magenraum verflüchtigt und dadurch das benötigte Volumen des Magenballons reduzieren würde.

US 4,485,805 beschreibt das Einbringen eines Magenballons durch die Speiseröhre, das Befüllen desselben mit Flüssigkeit und damit einen, im Magen freischwimmenden, mit Flüssigkeit gefüllten Magenballon. Röntgenmarkierungen zur Identifikation des Ballons im Magen sind in dieser Schrift ebenfalls beschrieben. Zum Befüllen des Ballons werden Wasser, Kochsalzlösung, gelartige Substanzen, Zellulose, also verschiedene, möglichst raumgreifende Medien empfohlen, die durch die Speiseröhre mittels Füllkatheter in den Magenballon eingebracht werden können. Bereits in dieser Schrift wird eingangs auf die Möglichkeit hingewiesen, den im Magen liegenden Ballon anstelle von Flüssigkeit mit Gas, vorzugsweise Luft zu füllen. Erwähnt werden auch die möglichen Probleme im Zusammenhang mit einem luftgefüllten, intragastralen Ballon, nämlich der relativ schnelle Verlust an Füllvolumen wegen Gas-Druckverlust durch die dünne elastische Hülle des Ballons, aber auch das Risiko eines gasgefüllten Ballons innerhalb des Körpers, bei Veränderungen des äußeren Luftdruckes, z.B. bei Reisen in großer Höhe, bei Flugreisen mit mangelhaftem Kabinendruckausgleich usw..

US 4,607,618 zeigt einen faltbaren Hohlkörper, welcher im gefalteten Zustand durch die Speiseröhre in den Magen vorgebracht werden kann. Anschließend wird der Hohlkörper im Magen aufgespannt, wodurch er die raumgreifende Form annimmt und dadurch das Magenvolumen reduziert wird. Aufgespannt wird der Hohlkörper über ein teilweise starres, im Inneren befindliches Gestänge. Ziel dieser Erfindung ist es, einen im Magen liegenden, raumgreifende Hohlkörper zur Verfügung zu stellen, welcher nicht unkontrolliert wegen eines plötzlichen Volumenverlustes (Undichtheit der Ballonwand) in den Darmtrakt abgeht und dort, für den Patienten zu gefährlichen Komplikationen führen könnte.

In US 5,234,454 wird ein Magenballon geoffenbart, welcher durch die Bauchdecke (perkutan, transkutan), mittels einem Katheter im Magen des Patienten platziert wird. Dabei kommt es zu einer Bildung einer dauerhaften Gastrostomie - einer permanenten Verbindung der Magenhöhle nach außen, zur Körperoberfläche hin. Diese direkte Verbindung durch den Katheter - auch Magensonde genannt - zum Magenballon im Inneren des Magens, soll eine schnelle Anpassung und einfache Überwachung des Füllvolumens des Ballons ermöglichen. Allerdings hat sich dieser Zugang, wohl wegen der permanenten Infektionsgefahr, die von einer solchen Verbindung eines Hohlorganes zur Körperoberfläche hin ausgeht, nicht durchgesetzt. Der Katheter, an dem der im Magen befindliche Ballon befestigt ist, wird durch einen, an der Durchtrittsstelle an der Mageninnenwand befindlichen Ringballon und einem, auf dem Katheter verschiebbaren Festellring auf der Außenseite, im Körpergewebe fixiert.

US 2006/0271088 A1 zeigt die Ausführung eines Magenballons, welcher mit endoskopischer Unterstützung perkutan im Magen platziert wird. Bei dieser Ausführung befindet sich der Magenballon am Ende eines flexiblen Katheters, welcher in die Magenhöhle hineinragt. Die Durchtrittsstelle des Katheters durch die Magenwand wird durch zwei, an die Magenwand innen und außen angedrückte Formstücke, Ringballone abgedichtet. Der Katheter selbst endet in mehreren, subkutan liegenden Kammern, über die das Füllvolumen des Magenballons aber auch der für die Katheterfixierung in der Magenwand benötigten Ringballone angepasst werden kann. Die geoffenbarte Durchführung des Katheters durch die Magenwand stellt ein permanentes Risiko für Durchtritt von Mageninhalt in die Bauchhöhle dar. Der im Magen schwimmende Magenballon, welcher sich am Ende des Katheters befindet, übt eine Dauerbelastung auf die Durchtrittsstelle des Katheters durch die Magenwand aus. Diese wiederum stellt ein hohes Gesundheitsrisiko für den Patienten dar.

Zusammengefasst weisen heute bekannte Magenballonsysteme folgende Schwachstellen auf, die eine Langzeitbehandlung von an Fettleibigkeit erkrankten Patienten nicht möglich machen.
● Ballone platzen unerwartet früh, gehen in den Darm ab, was in weiterer Folge zu einem Darmverschluss führen kann. Deshalb werden Magenballone 6 bis 9 Monate nach dem Einsetzen in den Magen wieder endoskopisch entfernt.
● Es kommt in wenigen Fällen sowohl beim endoskopischen Einbringen, als auch beim Entfernen von Magenballonsystemen zu Verletzungen an der Speiseröhre des Patienten.
● Einrichtungen, die nicht ein dauerhafte Behandlung der Fettleibigkeit ermöglichen, die nach 6 bis 9 Monaten wieder entfernt werden müssen, sind zur Behandlung der krankhaften Fettleibigkeit nicht geeignet, da Patienten nach Entfernen des Magenballons sofort wieder an Gewicht zunehmen.
● Flüssigkeitsgefüllte Magenballone führen auf Dauer auf Grund des Eigengewichtes zu einer Längsstreckung des Magens, also zu einer ungewollten Vergrößerung des Magenvolumens. Auch das ist mit ein Grund, weshalb heute bekannte Systeme nach kurzer Verweilzeit im Magen wieder entfernt werden.
● Die heute bekannten gasgefüllten Magenballone können die Anforderungen an eine Langzeitbehandlung der krankhaften Fettleibigkeit noch nicht erfüllen. Unerwarteter plötzlicher Gasverlust und der damit verbundene Abgang in den Darmtrakt, kann schwere Komplikationen für den Patienten zur Folge haben.
● Magenballone mit einer Katheterverbindung durch die Bauchdecke nach außen an die Körperoberfläche, sind für eine Langzeitverweildauer von mehreren Jahren wegen der auf Dauer zunehmenden Infektionsgefahr völlig ungeeignet.

Ziel der Erfindung ist es, eine magenvolumenreduzierende Einrichtung zur Verfügung zu stellen, welche eine unbegrenzte Langzeitbehandlung der krankhaften Fettleibigkeit ermöglicht. Die Einrichtung muss über die gesamte Nutzungsdauer auf die wechselnden Patientenbedürfnisse einstellbar sein. Im Falle von auftretenden technischen Schwächen - z.B. Materialversagen bestimmter Bauteile - muss eine einfache, den Patienten nicht belastende Korrektur der Einrichtung möglich sein. Die Möglichkeit der Überwachung mittels Gastroskopie, Röntgen, CT, MRI oder aber auch Ultraschall, gibt dem Patienten und dem behandelnden Arzt die notwendige Kontrollmöglichkeit, über die Funktionsweise und den Zustand der Einrichtung während der gesamten Nutzungsdauer. Erfindungsgemäß gelingt dies durch eine Einrichtung mit den Merkmalen des Anspruch 1.

Bei der Einrichtung handelt es sich um eine chirurgisch implantierbare Einrichtung, bei welcher die verschlossene Zugangsöffnung zum Hohlraum der Einrichtung unterhalb der Haut des Patienten liegt. Die Zugangsöffnung zum Hohlraum der Einrichtung befindet sich an einem Tubus, dessen anderes Ende in die Magenhöhle hineinragt und an dem die raumgreifenden Hüllen befestigt sind. Der Tubus wird vorzugsweise aus einem nicht magnetisierbaren, aber bioinerten Material wie z.B. Titanium gefertigt, dessen Flächen, die im Inneren des Magens in Kontakt mit Magensäure stehen, zur Erhöhung der Widerstandfähigkeit vergoldet sein können. Die Fertigung des Tubus aus bioinerten Kunststoffen wie z.B. PEEK, Polysulfon, Polypropylen, Nylon mit oder ohne Beschichtung ist ebenfalls denkbar.

Um die Durchtrittsstelle des Tubus durch die Magenwand gegen Austritt von Mageninhalt in die Bauchhöhle zu sichern, ist am Tubus ein chirurgisches Netz fest angebracht, wie es zur Verstärkung von weichem Gewebe, z. B. in der Chirurgie von Leistenbrüchen, heute standardmäßig eingesetzt wird. Auf dem im Magen liegenden Ende des Tubus ist ein aufblasbarer Ringballon integriert, welcher dafür sorgt, dass die Mageninnenwand (Mukosa) mit leichtem Druck an die Bauchdecke gedrückt wird und die Durchtrittsstelle des Tubus durch die Magenwand von den Bewegungen des Mageninhaltes, die auf das System wirken, entlastet wird. Das am Tubus befestigte Netz wird mit der äußeren Gewebelage des Magens (Serosa) und der posterioren Faszie der Bauchdecke vernäht. Damit wächst die Magenwand an die Bauchdecke an und der Durchtrittsstelle des Tubus in das Innere des Magens wird ein Austreten von Mageninhalt in die Bauchhöhle sicher und auf Dauer verhindert.

Die am Tubus befestigten, raumgreifenden Hüllen der Einrichtung, die in das Mageninnere hineinragen, können aus verschiedenen Materialien gefertigt sein. Bei der Materialwahl ist zu beachten, dass die gewählten Materialen gegenüber den im Magen vorkommenden Flüssigkeiten widerstandsfähig, luftdicht, flüssigkeitsdicht, leicht faltbar oder aber flexibel sind. Dafür kommen sowohl textilverstärkte Kunststofffolien, beschichtete Metallfolien, Folien aus bioinerten Elastomeren wie Silikon oder Polyurethan, usw. in Frage. Sowohl die Anzahl der am Tubus angebrachten raumgreifenden Hüllen als auch die Reihenfolge, in welcher die verschiedenen Materialien angebracht sind, kann unterschiedlich sein. Eine mögliche Ausführung besteht z.B. aus 5 raumgreifenden Hüllen, die ineinander angeordnet in die Magenhöhle hineinragen, wobei die äußerste Hülle aus einem textilverstärkten Silikon, die zweite Hülle aus einer gasdichten Folie aus Nylon, die dritte Hülle wiederum aus dem Material der äußeren Hülle besteht, die vierte Hülle aus einem Textilgewebe wie Seide besteht und die innerste fünfte Hülle aus einer Folie aus vergoldetem Aluminium besteht. Erfindungsgemäß kann die geometrische Form der raumgreifenden Hüllen an die Anatomie der Magenhöhle angepasst werden.

Die Erfindung sieht vor, dass die im Mageninneren raumgreifenden Hüllen intraoperativ durch den offenen Tubus mit kleinen, leichten Füllkörpern, wie z.B. Silikon-, Polyurethan-, Zucker- oder Zellulosekörpern gefüllt werden. Dadurch entsteht ein raumgreifendes, geschlossenes, druckloses Gebilde in der Magenhöhle, welches die Menge der Nahrungsaufnahme pro Mahlzeit einschränkt, wodurch der gewünschte Gewichtsverlust erzielt wird. Dieses Gebilde innerhalb des Magens übt keinen Druck auf die Magenschleimhaut aus, eine Voraussetzung dafür, dass das System für die Langzeitanwendung geeignet ist. Nach dem intraoperativen Befüllen der raumgreifenden Hüllen mit Füllkörpern, wird der Tubus verschlossen und die darüber liegenden Gewebeschichten, wie subkutanes Fettgewebe und die Haut, werden mit einer chirurgischen Naht verschlossen.

Ein weiteres, erfinderisches Merkmal der Einrichtung, bildet der in den Tubusverschluss integrierte Zugang zum Inneren der raumgreifenden Hüllen. Damit wird eine wichtige Forderung - nämlich die Anpassung des raumgreifenden Volumens der Hüllen in der Magenhöhle an die Erfordernisse des Patienten im Zusammenhang mit der erzielten Gewichtsabnahme über einen unbeschränkten Zeitraum - möglich. Erfindungsgemäß ist dafür im Tubusverschluss eine dicke Scheibe aus Silikon integriert, durch welche der behandelnde Arzt, transkutan mittels Spritze die raumgreifenden Hüllen zusätzlich mit Luft oder geeigneter Flüssigkeit befüllen kann. Sollte weniger raumgreifendes Volumen der im Magen befindlichen Hüllen gewünscht sein, kann über denselben Zugang, transkutan mittels Spritze Flüssigkeit oder Luft aus den Hüllen abgesaugt werden. Erfindungsgemäß werden in den Zwischenräumen der raumgreifenden Hüllen besondere Marker eingebracht, damit der Patient bemerkt, wenn z.B. die äußerste Hülle durch die mechanische Belastung, ausgelöst durch Speisebrei und die Peristaltik des Magens, zerstört wurde. In einem Ausführungsbeispiel könnte z.B. der Zwischenraum der raumgreifenden Hüllen mit Farbstoff gefüllt sein, welcher vom Patienten bei der nächsten Entleerung des Darmes registriert werden würde. Kleine, hell gefärbte, aus leichtem Kunststoff gefertigte Kugeln sind als Marker ebenfalls denkbar.

Erfindungsgemäß handelt es sich um eine Einrichtung zur dauerhaften Behandlung der Fettleibigkeit. Dies bedeutet, die Einrichtung ist so ausgelegt, dass sie den Patienten bis an sein Lebensende begleiten kann. Sowohl andauernde Bewegung des Magens zum Austreiben des Speisebreis (Peristaltik), als auch mechanische Belastung auf die in der Magenhöhle befindlichen raumgreifenden Hüllen durch den Speisebrei selbst (kann harte und scharfkantige Bestandteile, z.B. Knochensplitter, Fischgräte usw. enthalten), führen früher oder später zu einer Beschädigung der raumgreifenden Hüllen, was einen Austausch derselben notwendig macht. Erfindungsgemäß stellt ein solcher Austausch einen minimal invasiven chirurgischen Eingriff dar, welcher unter lokaler Betäubung durchgeführt werden kann. Die Haut über dem subkutanen Tubusverschluss wird eröffnet, der Tubusverschluss vom Chirurgen freigelegt und durch Drehen geöffnet, falls notwendig werden die sich noch in der letzten intakten Hülle befindlichen Füllkörper abgesaugt, die Reste der gebrochenen Hüllköper entfernt und ein neuer Satz Hüllkörper in der Tubus eingesetzt. Die innerste raumgreifende Hülle wir mit frischen Füllkörpern gefüllt und der Tubus anschließend verschlossen. Die Hautinzision über dem Tubus wird chirurgisch versorgt und das System ist für den nächsten Langzeitzyklus - der mehrere Jahre dauern kann - wieder einsatzbereit.

Erfindungsgemäß kann nach derselben Vorgangsweise auch auf einfache und minimal invasive Art und Weise auf Krankheitsbilder des Magens, wie z.B. Auftreten eines Magengeschwürs, die ein temporäres Entfernen der raumgreifenden Hüllen aus dem Mageninneren notwendig machen, reagiert werden.

Erfindungsgemäß erlaubt der Innendurchmesser des in der Magenwand fixierten Tubus nach Entfernen der raumgreifenden Hüllen auch einen Zugang zu Magenhöhle für chirurgische, diagnostische Behandlungen der Magenhöhle und des oberen Gastrointestinaltraktes.

Vorteile und Einzelheiten der Erfindung werden anhand der beiliegenden Zeichnungen an einem möglichen Ausführungsbeispiel erläutert.

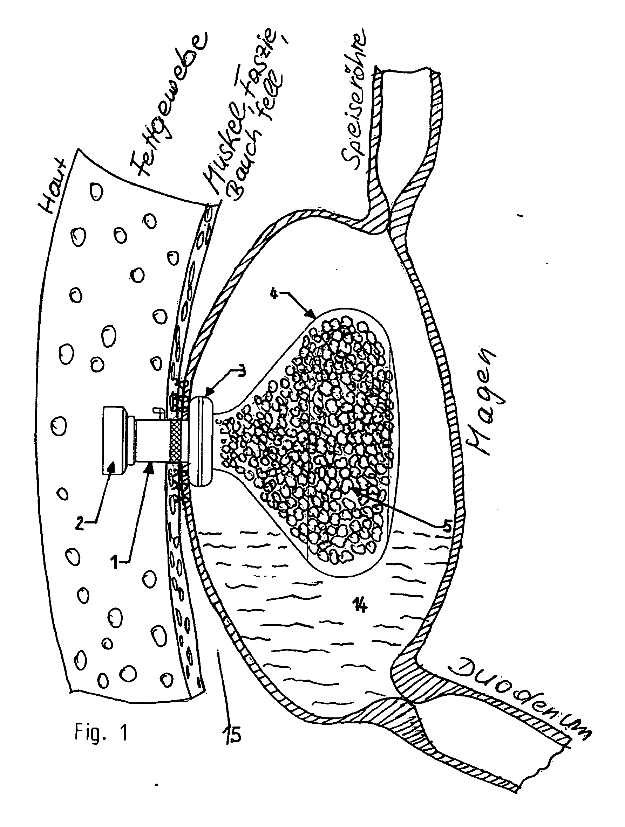

Fig 1 Zeigt die implantierbare, magenvolumenreduzierende Einrichtung im implantierten Zustand.

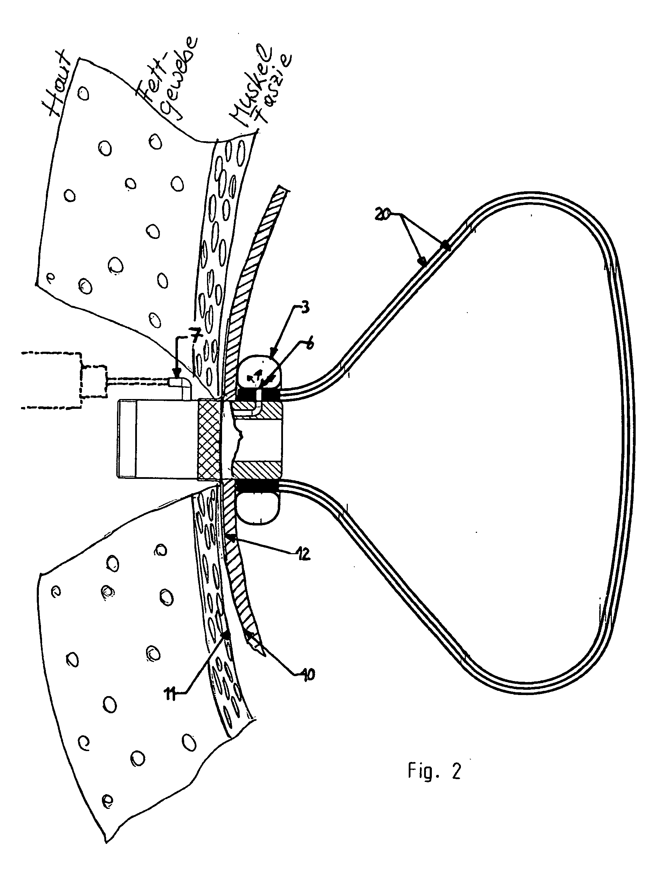

Fig 2 Zeigt das Befüllen des Ringballons, welcher sich auf dem Tubus befindet und diesen in der Durchtrittsstelle durch die Magenwand stabilisiert und gegen Herausrutschen aus dem Magen sichert. Nach dem Befüllen des Ringballons mittels Spritze und Flüssigkeit, z.B. sterilem Wasser, wird der flexible Anschlusskatheter mit einem chirurgischen Clip oder aber mit einer Fadenligatur verschlossen.

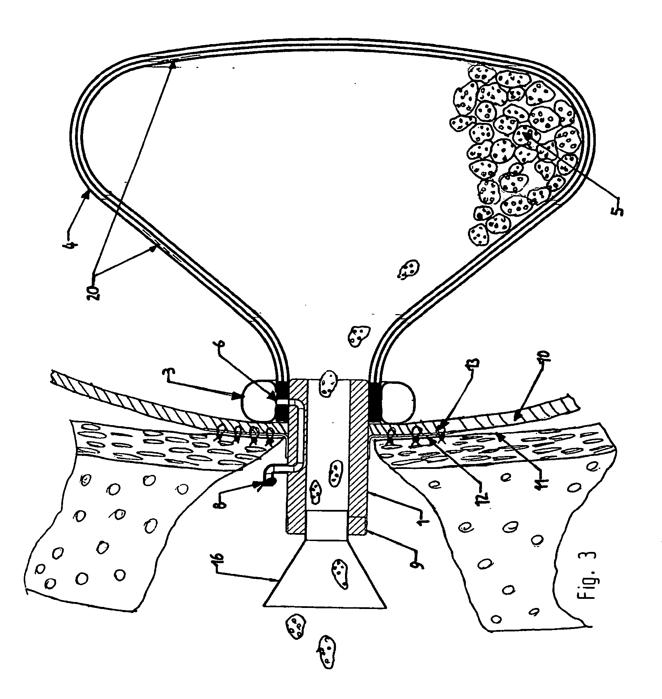

Fig 3 Nach dem Befüllen des Ringballons wird der Tubus an die Mageninnenwand gezogen und das am Tubus angebrachte chirurgische Netz mit der äußeren Magenwand und der posterioren Faszie der Bauchdecke angenäht. Damit ist der Tubus in seiner Lage gegen axiale Längsverschiebungen gesichert und die Tubusdurchtrittsstelle durch die Magenwand abgedichtet. Anschließend werden die raumgreifenden Hüllen durch den offenen Tubus mit den entsprechenden Füllkörpern gefüllt.

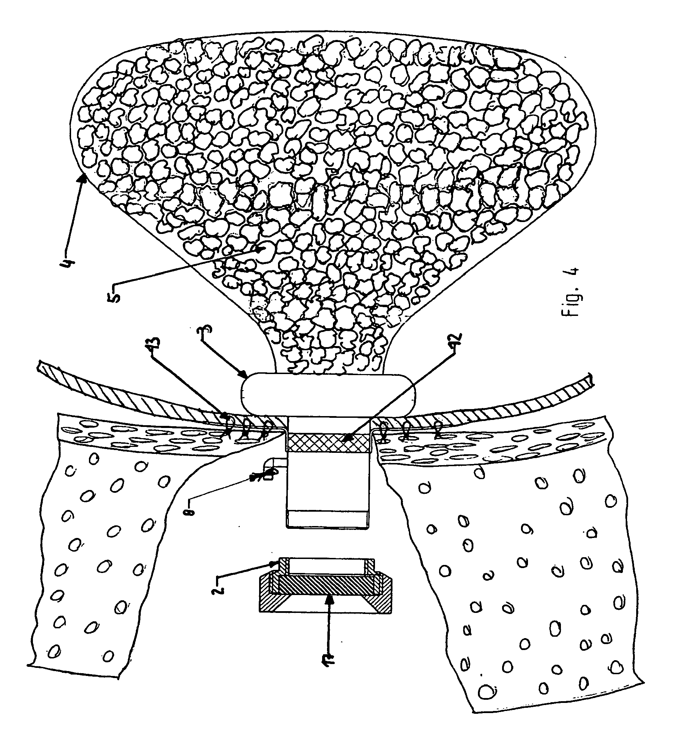

Fig 4 Zeigt die mit Füllkörpern gefüllte innere raumgreifende Hülle vor dem Aufschrauben des Tubusverschlussdeckels mit integrierter Portscheibe aus Silikon.

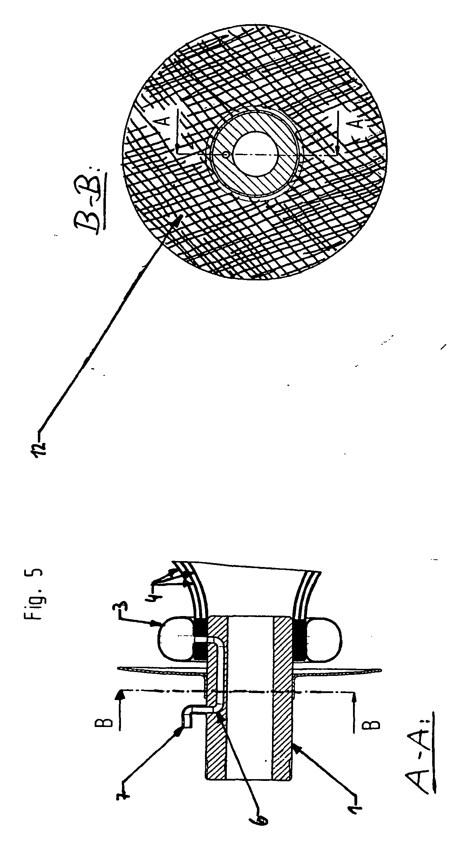

Fig 5 Zeigt eine mögliche Ausführungsform des Tubus mit auf dem im Magen liegenden Ende aufgeklebten 3 Lagen von raumgreifenden Hüllen, mit dem am selben Ende aufgeklebten Ringballon mit Füllkanal und flexiblem Anschlusskatheter, sowie dem am Umfang des Tubus fest angebrachten, kreisförmigen chirurgischen Netz. Das andere Ende des Tubus ist für die Aufnahme des Verschlussdeckels mit einem Gewinde versehen.

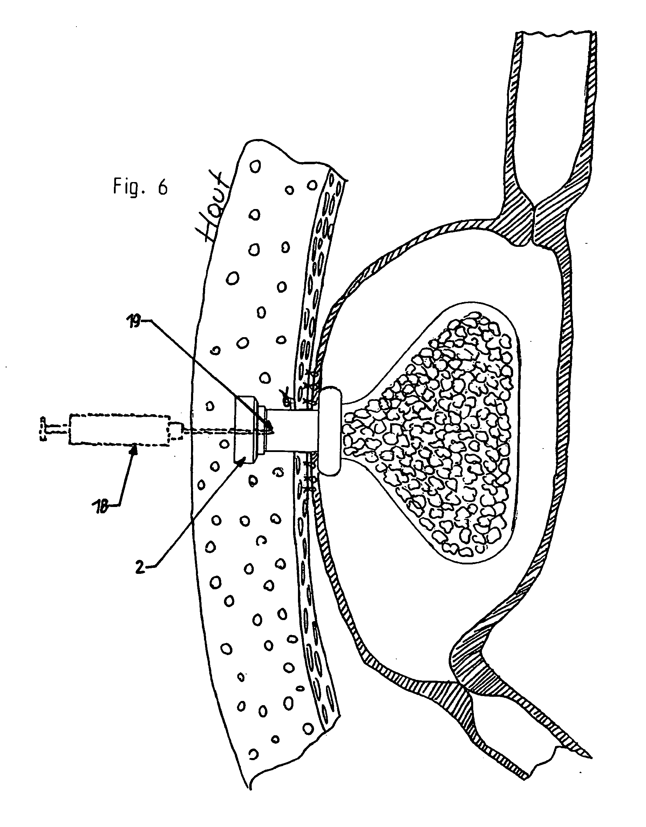

Fig 6 Zeigt das Anpassen des Füllvolumens der im Magen liegenden raumgreifenden Hüllen. Dabei wird mittels Spritze perkutan die Silikonscheibe im Verschlussdeckel des Tubus durchstochen und mittels Flüssigkeit oder aber Luft das Füllvolumen der Hüllen angepasst.

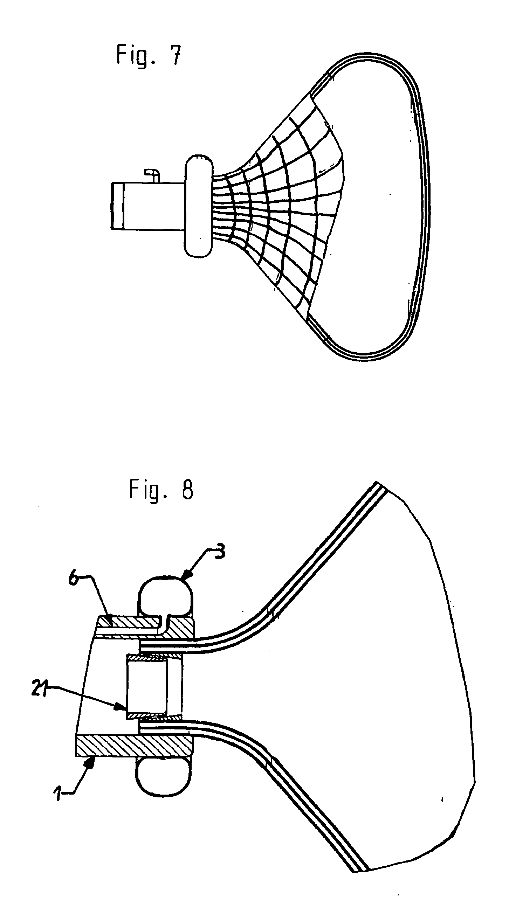

Fig 7 Zeigt eine mögliche Ausführungsform der raumgreifenden Hüllen, bei der das am Tubus angeschlossene Endstück durch eine in die Hülle integrierte Netzstruktur verstärkt ist. Die Filamente der eingearbeiteten Netzstruktur können aus verschiedenen Materialien, wie Kunststofffasern, Naturfasern, Metallfäden, vorzugsweise aus bioinertem Polypropylen oder Polyester bestehen.
Fig 8 Zeigt eine mögliche Ausführungsform der Befestigung der ineinanderliegenden, raumgreifenden Hüllen am Tubus mittels innenliegendem Klemmring.

Ein Ausführungsbeispiel der erfindungsgemäßen Einrichtung ist in den Figuren 1 bis 8 gezeigt.

Die Einrichtung besteht aus einem Tubus 1, an dessen einem Ende zur Aufnahme des Verschlussdeckels 2 ein Gewinde 9 angebracht ist, und an dessen anderem Ende ein Ringballon 3 und die raumgreifenden Hüllen 4 angebracht sind. Die in das Mageninnere reichenden raumgreifenden Hüllen 4 werden mit leichten Füllkörpern 5 und falls benötigt zusätzlich mit einer Flüssigkeit oder einem Gas gefüllt. Als Füllkörper 5 könnten z.B. kleine Schaumstoffkugeln aus Silikon oder Polyurethan verwendet werden. Jedes andere, nicht toxische, leichte und abriebfeste Material kann ebenfalls als Füllkörper für die raumgreifenden Hüllen dienen.

Der Ringballon 3 wird über einen in der Tubuswand verlaufenden Füllkanal 6 mit Flüssigkeit, z.B. einem Röntgenkontrastmittel, sterilem Wasser, Kochsalzlösung oder ähnlichem mittels Spritze gefüllt. Der flexible Anschlusskatheter 7 wird nach dem Befüllen des Ringballons 3 mit einer Fadenligatur 8 oder einem chirurgischen Clip verschlossen. Nun wird der Tubus 1 mit dem gefüllten Ringballon 3 nach ventral - zur Körpervorderseite hin - gezogen, damit die äußere Magenwand 10 an der Innenwand der Bauchdecke 11 anliegt. Der Chirurg vernäht nun das am Tubus festgemachte zirkuläre Netz 12 mit der äußeren Magenwand 10 und der posterioren Faszie der Bauchdecke 11 mittels chirurgischer Naht 13.

Damit ist der Tubus 1 in axialer Längsrichtung im Gewebe fixiert, die Tubusdurchtrittsstelle durch die Magenwand mittels Ringballon 3 abgedichtet und entlastet und das chirurgische Vernähen des zirkulären Netzes 12 zwischen der posterioren Faszie der Bauchwand 11 und der äußeren Magenwand 10, sorgt dafür, dass diese Gewebelagen miteinander verwachsen und die Tubuseintrittsstelle in den Magen auf Dauer verschlossen und ein Austritt von Mageninhalt 14 in die Bauchhöhle 15 auf Dauer sicher verhindert wird. Das zirkuläre Netz 12 kann in weiteren Ausführungen jegliche Form einnehmen, sei es quadratisch, rechteckig, oval oder aber der Umfang und die Größe vom Chirurgen während der Operation willkürlich, nach Bedarf mittels Schere zugeschnitten werden.

Zum Befüllen der raumgreifenden Hüllen 4 mit den Füllkörpern 5 kann ein steriler Trichter 16 auf das offene Ende des Tubus 1 gesetzt werden.

Nach dem Befüllen der raumgreifenden Hüllen 4 mit den Füllkörpern 5 wird der Tubus 1 durch Aufschrauben des Verschlussdeckels 2, mit integrierter Portscheibe 17 aus Silikon, sicher verschlossen.

In dem in Figur 5 gezeigten Ausführungsbeispiel bestehen die raumgreifenden Hüllen 4 aus drei Lagen, wobei die Materialen der Hüllen unterschiedlich sein können. So können die raumgreifenden Hüllen z.B. aus einer Kombination von flüssigkeitsdichten, gasdichten, besonders abriebfesten, besonders reißfesten und/oder flexiblen Materialien bestehen. Bevorzugt wird eine Kombination aus einer beschichteten, reiß- und abriebfesten Hülle und einer gasdichten und einer flüssigkeitsdichten Hülle gewählt. Die Anzahl der Lagen der raumgreifenden Hülle kann 1 bis ein Vielfaches davon betragen, bevorzugt werden drei bis fünf ineinander-liegende, raumgreifende Hüllen 4 am Tubusende befestigt.

Erfindungsgemäß ist die Einrichtung als Langzeitimplantat konzipiert. In Abhängigkeit der erzielten Gewichtsabnahme des Patienten, wird in unregelmäßigen Abständen eine Einstellung des Volumens innerhalb der im Mageninneren befindlichen raumgreifenden Hüllen notwendig. Das kann sowohl eine Zunahme des Volumens - z.B. bei zu geringem Gewichtsverlust des Patienten - als auch eine Reduzierung des Volumens - z.B. bei zu hohem Gewichtsverlust des Patienten - bedeuten.

Diese Volumeneinstellung der raumgreifenden Hüllen geschieht wie in Fig. 6 gezeigt, mittels Spritze 18 und perkutanes Durchstechen der im Verschlussdeckel 2 integrierten Portscheibe aus Silikon 17. Nun kann über die - in den von den raumgreifenden Hüllen geschaffenen Hohlraum - hineinragende Nadel 19 der Spritze das Volumen mittels Einbringen oder Absaugen von Flüssigkeit und oder Luft an die Bedürfnisse des Patienten angepasst werden.

### Legende zu den Hinweisziffern:

- 1: Tubus
- 2: Verschlussdeckel
- 3: Ringballon
- 4: Raumgreifende Hülle
- 5: Füllkörper
- 6: Füllkanal
- 7: Anschlusskatheter
- 8: Fadenligatur
- 9: Gewinde
- 10: Magenwand
- 11: Bauchwand
- 12: Zirkuläres Netz
- 13: Chirurgische Naht
- 14: Mageninhalt
- 15: Bauchhöhle
- 16: Trichter
- 17: Portscheibe
- 18: Spritze
- 19: Nadelspitze
- 20: Marker
- 21: Klemmring

## Patentansprüche

1. Eine chirurgische Einrichtung, die als Langzeitimplantat die Behandlung der Fettleibigkeit beim Menschen ermöglicht, **dadurch gekennzeichnet, dass** ein Tubus (1), dessen in Gebrauch unterhalb der Haut liegendes Ende mit einem wiederholt zu öffnenden Verschlussdeckel (2) mit integrierter Portscheibe (17) versehen ist, bis in die Magenhöhle hineinragt und am in der Magenhöhle liegenden Ende des Tubus umfänglich mehrere Schichten aus raumgreifenden, in den Magen hineinragenden geschlossenen Hüllen (4) angebracht sind, die mit Füllkörpern (5) gefüllt sind, wobei der Tubus (1) selbst im Gewebe durch eine Kombination von einem im Mageninneren liegenden Ringballon (3) und einem mit Magenwand und Bauchdecke chirurgisch vernähten, fest mit dem Tubus verbundenen Netz (12), gegen axiale Verschiebungen in Längsrichtung des Tubus (1) gesichert ist.

2. Chirurgische Einrichtung nach Anspruch 1, **dadurch gekennzeichnet, dass** die im Verschlussdeckel (2) integrierte Portscheibe (17) aus Silikon auch nach vielfachem Durchstechen mit einer Nadel (18) das Innenvolumen der raumgreifenden Hüllen (4) sicher gegenüber dem subkutanen Fettgewebe des Patienten abdichtet.

3. Chirurgische Einrichtung nach Anspruch 1, **dadurch gekennzeichnet, dass** die Materialien der einzelnen Hüllen (4) unterschiedlich sind.

4. Chirurgische Einrichtung nach Anspruch 1, **dadurch gekennzeichnet, dass** eine Kombination an ineinander liegenden, raumgreifenden Hüllen (4), zumindest aus je einer reiß- und abriebfesten Hülle, einer gasdichten Hülle und einer flüssigkeitsdichten Hülle besteht.

5. Chirurgische Einrichtung nach Anspruch 1, **dadurch gekennzeichnet, dass** mindestens eine raumgreifende Hülle (4) bis ein Vielfaches von einer Hülle am in das Mageninnere hineinragenden Tubus (1) befestigt ist.

6. Chirurgische Einrichtung nach Anspruch 1, **dadurch gekennzeichnet, dass** am in das Mageninnere ragenden Ende des Tubus (1) ein Ringballon (3) angebracht ist, welcher durch einen in der Tubuswand verlaufenden Füllkanal (6) und einem am Tubus befindlichen Anschlusskatheter (7) mit Flüssigkeit oder Gas befüllt werden kann.

7. Chirurgische Einrichtung nach Anspruch 1, **dadurch gekennzeichnet, dass** die in das Mageninnere (14) ragenden raumgreifenden Hüllen (4) über den Tubus (1) und einem aufgesetztem Trichter (16) mit Füllkörpern (5) befüllt werden können.

8. Chirurgische Einrichtung nach Anspruch 1, **dadurch gekennzeichnet, dass** die Füllkörper (5) aus einem abriebfesten Material und mit einem spezifischen Gewicht, das leichter als Wasser ist, bestehen, sowie in unterschiedlicher geometrischer Form ausgeführt sind, deren Größe allerdings ein sicheres, natürliches Ausscheiden aus dem Körper durch den Darmtrakt des Patienten ermöglicht.

9. Chirurgische Einrichtung nach Anspruch 1, **dadurch gekennzeichnet, dass** die Füllkörper (5) aus Silikon- oder Polyurethanschaumstoff bestehen.

10. Chirurgische Einrichtung nach Anspruch 1, **dadurch gekennzeichnet, dass** der Tubus (1) mittels Verschlussdeckel (2) geschlossen ist, welcher durch ein Gewinde (9) oder aber einen Bajonettverschluss mehrfach, wiederholbar geöffnet oder geschlossen werden kann.

11. Chirurgische Einrichtung nach Anspruch 1, **dadurch gekennzeichnet, dass** zwischen den verschiedenen Schichten der raumgreifenden Hüllen (4) Marker (20) eingelegt sind, die nach dem Undichtwerden der jeweils äußeren raumgreifenden Hülle (4) durch den Darm des Patienten abgehen und vom Patienten im Stuhl bemerkt werden können, die aber auch bei einer Röntgenkontrolle der chirurgischen Einrichtung bemerkt werden, wodurch die Unversehrtheit und die korrekte Lage der raumgreifenden Hüllen (4) im Magen beurteilt werden kann.

12. Chirurgische Einrichtung nach Anspruch 1, **dadurch gekennzeichnet, dass** die Marker (20) aus lebensmittelechten, farbigen Substanzen bestehen, die zu einer besonderen Einfärbung des Stuhls führen.

13. Chirurgische Einrichtung nach Anspruch 1, **dadurch gekennzeichnet, dass** die Marker (20) aus farbigen, kleinen Körpern bestehen, die vom Patenten im Stuhl gesehen werden können.

14. Chirurgische Einrichtung nach Anspruch 1, **dadurch gekennzeichnet, dass** die raumgreifenden Hüllen (4) am Tubus (1) austauschbar mittels Klemmring (21) angebracht sind.

## Claims

1. A surgical device enabling as a long-term implant the treatment of obesity in humans, **characterised in that** a tube (1), whose end lying below the skin during use is provided with a cover lid (2) which has an integrated port disc (17) and is to be opened repeatedly, projects right into the stomach cavity and at the tube end lying in the stomach cavity there are circumferentially attached a plurality of layers of closed, space-consuming casings (4) projecting into the stomach, which casings are filled with filling bodies (5), wherein the tube (1) itself is, by a combination of an annular balloon (3) lying in the stomach interior and a mesh (12) which is surgically sutured to stomach wall and abdominal wall and is securely connected to the tube (1), secured in the tissue against axial displacements in the longitudinal direction of the tube (1).

2. A surgical device according to claim 1, **characterised in that** the silicone port disc (17) integrated in the cover lid (2) seals the inner volume of the space-consuming casings (4) securely with respect to the subcutaneous adipose tissue of the patient, even after multiple puncturings with a needle (18).

3. A surgical device according to claim 1, **characterised in that** the materials of the individual casings (4) are different.

4. A surgical device according to claim 1, **characterised in that** a combination of space-consuming casings (4) lying inside one another at least consists of one each of a tear-proof and abrasion-proof casing, a gas-tight casing and a fluid-tight casing.

5. A surgical device according to claim 1, **characterised in that** from at least one space-consuming casing (4) up to multiple casings is secured to the tube (1) projecting into the stomach interior.

6. A surgical device according to claim 1, **characterised in that** an annular balloon (3) is attached to that end of the tube (1) which projects into the stomach interior, which annular balloon (3) can be filled with fluid or gas by a filling channel (6) running in the tube wall and by a connection catheter (7) located at the tube.

7. A surgical device according to claim 1, **characterised in that** the space-consuming casings (4) projecting into the stomach interior (14) can be filled with filling bodies (5) via the tube (1) and a mounted funnel (16).

8. A surgical device according to claim 1, **characterised in that** the filling bodies (5) are composed of an abrasion-proof material and have a specific weight lighter than water and are designed in a different geometric form whose size though enables a reliable, natural discharge from the body through the patient's intestinal tract.

9. A surgical device according to claim 1, **characterised in that** the filling bodies (5) are composed of silicone foam or polyurethane foam.

10. A surgical device according to claim 1, **characterised in that** the tube (1) is closed by means of a cover lid (2) which can be opened or closed many times and repeatedly by a thread (9) or else a bayonet fixing.

11. A surgical device according to claim 1, **characterised in that** markers (20) are inserted between the different layers of the space-consuming casings (4), which markers (20) are evacuated through the patient's intestine after the leakage of the respective outer, space-consuming casing (4) and can be noticed in the faeces by the patient, which markers are however also perceived during an X-ray check of the surgical device, whereby the intactness and the correct position of the space-consuming casings (4) in the stomach can be judged.

12. A surgical device according to claim 1, **characterized in that** the markers (20) consist of food-safe, coloured substances which lead to a particular colouring of the faeces.

13. A surgical device according to claim 1, **characterised in that** the markers (20) consist of coloured, small bodies which can be seen in the faeces by the patient.

14. A surgical device according to claim 1, **characterised in that** the space-consuming casings (4) are attached to the tube (1) in an exchangeable manner by means of the clamping ring (21).

## Revendications

1. Dispositif chirurgical réalisé sous la forme d'un implant de longue durée et permettant le traitement de l'obésité chez l'homme,
dispositif **caractérisé en ce qu'**
il comporte un tube (1) dont l'extrémité située sous la peau en position d'utilisation est équipée d'un couvercle de fermeture (2) ayant un disque d'accès (17), intégré, pouvant être ouvert de façon répétée, qui pénètre dans la cavité gastrique et qui est équipé à son extrémité située dans la cavité gastrique, de manière volumineuse, de plusieurs couches d'enveloppes fermées (4) occupant l'espace pénétrant dans l'estomac qui sont remplies de corps de remplissage (5), le tube (1) étant lui-même bloqué dans le tissu vis-à-vis de déplacements axiaux dans sa direction longitudinale par la combinaison d'un ballon annulaire (3) situé à la partie interne de l'estomac et d'un filet ou d'un treillis (12) fixé au tube (1), et cousu chirurgicalement avec la paroi de l'estomac et la paroi abdominale.

2. Dispositif chirurgical conforme à la revendication 1,
**caractérisé en ce que**
le disque d'accès (17) intégré dans le couvercle de fermeture (2), est réalisé en silicone et garantit de façon sûre, l'étanchéité du volume interne des enveloppes (4) occupant l'espace par rapport au tissu gras sous-cutané du patient, également après plusieurs perforations avec une aiguille (18).

3. Dispositif chirurgical conforme à la revendication 1,
**caractérisé en ce que**
les matériaux des différentes enveloppes (4) sont différents.

4. Dispositif chirurgical conforme à la revendication 1,
**caractérisé en ce que**
la combinaison d'enveloppes (4) occupant l'espace, situées les unes dans les autres, consiste en au moins respectivement une enveloppe résistant à la déchirure et à l'arrachement, une enveloppe étanche aux gaz et une enveloppe étanche aux liquides.

5. Dispositif chirurgical conforme à la revendication 1,
**caractérisé en ce qu'**
au moins une enveloppe (4) occupant l'espace ou plusieurs de ces enveloppes est (sont) fixée(s) au tube (1) pénétrant à la partie interne de l'estomac.

6. Dispositif chirurgical conforme à la revendication 1,
**caractérisé en ce qu'**
à l'extrémité du tube (1) pénétrant à l'intérieur de l'estomac, est monté un ballon annulaire (3) pouvant être rempli de liquide ou de gaz par un canal de remplissage (6) s'étendant dans la paroi du tube et un cathéter de liaison (7) situé sur le tube.

7. Dispositif chirurgical conforme à la revendication 1,
**caractérisé en ce que**
les enveloppes (4) occupant l'espace pénétrant à l'intérieur de l'estomac (14) peuvent être remplies de corps de remplissage (5) par le tube (1) et un entonnoir (16) monté sur celui-ci.

8. Dispositif chirurgical conforme à la revendication 1,
**caractérisé en ce que**
les corps de remplissage (5) sont constitués d'un matériau résistant à la déchirure et ayant un poids spécifique inférieur à celui de l'eau et sont réalisés avec différentes formes géométriques dont la dimension permet cependant une élimination naturelle sure du corps par le tractus intestinal du patient.

9. Dispositif chirurgical conforme à la revendication 1,
**caractérisé en ce que**
les corps de remplissage (5) sont réalisés en mousse de silicone ou en mousse de polyuréthane.

10. Dispositif chirurgical conforme à la revendication 1,
**caractérisé en ce que**
le tube (1) est fermé par un couvercle de fermeture (2) qui peut être ouvert ou fermé de façon répétitive par un filetage (9) ou toutefois une fermeture à baïonnette.

11. Dispositif chirurgical conforme à la revendication 1,
**caractérisé en ce qu'**
entre les différentes couches d'enveloppes (4) occupant l'espace, sont insérés des repères (20) pouvant être éliminés par l'intestin des patients après que l'enveloppes (4) occupant l'espace respective externe, soient devenue perméable, et être remarqués par les patients dans les selles, mais qui sont toutefois également remarqués lors d'un contrôle aux rayons X du dispositif chirurgical, permettant ainsi de déterminer l'intégrité et la position correcte des enveloppes (4) occupant l'espace dans l'intestin.

12. Dispositif chirurgical conforme à la revendication 1,
**caractérisé en ce que**
les repères (20) sont réalisés en des substances colorées de pureté alimentaire qui entraînent une coloration particulière des selles.

13. Dispositif chirurgical conforme à la revendication 1,
**caractérisé en ce que**
les repères (20) sont constitués par de petits corps colorés qui peuvent être visualisés par les patients dans les selles.

14. Dispositif chirurgical conforme à la revendication 1,
**caractérisé en ce que**
les enveloppes (4) occupant l'espace sont montées de manière interchangeable sur le tube (1) au moyen d'une bague de serrage (21).
